# EUROPEAN PATENT APPLICATION

(11) **EP 1 533 289 A1**
(43) Date of publication of application: **25.05.2005**
(21) Application number: 03766655.9
(22) Date of filing: 29.07.2003
(51) Int. Cl.: C07C 13/567, C07C 13/66, C09K 11/06, H05B 33/14, H05B 33/22

(54) **ANTHRACENE DERIVATIVES AND ORGANIC ELECTROLUMINESCENT DEVICES MADE BY USING THE SAME**

(30) Priority: 02.08.2002 JP 2002225636
(71) Applicant: Idemitsu Kosan Co., Ltd., Tokyo 100-8321 (JP)
(72) Inventor: IDO, Motohisa, Sodegaura-shi, Chiba 299-0205 (JP); FUNAHASHI, Masakazu, Sodegaura-shi, Chiba 299-0205 (JP); TOKAIRIN, Hiroshi, Sodegaura-shi, Chiba 299-0205 (JP)
(74) Representative: Gille Hrabal Struck Neidlein Prop Roos
(86) International application number: PCT/JP2003/009606
(87) International publication number: WO 2004/013073

(57) **Abstract**

An anthracene derivative represented by the following general formula (1) which enables an organic electroluminescence device to exhibit a great efficiency of light emission and uniform light emission even at high temperatures since crystallization is suppressed and no thermal decomposition takes place during vapor deposition and an organic electroluminescence device utilizing the derivative, are provided. 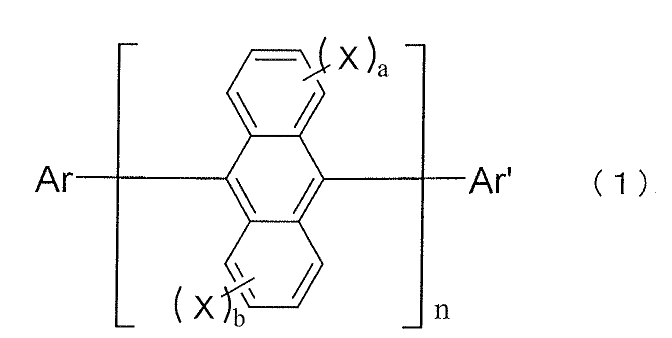 [Ar represents a group represented by the following general formula (2): 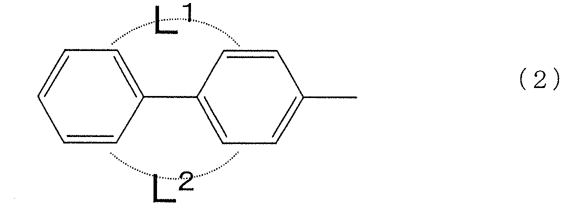 (L¹ and L² each represent a substituted or unsubstituted methylene group, ethylene group or the like, and at least one of them is present), Ar' represents a substituted or unsubstituted aryl group having 6 to 50 nuclear carbon atoms, X represent an alkyl group or the like, a and b each represent an integer of 0 to 4, and n represents an integer of 1 to 3.]

## Description

### TECHNICAL FIELD

The present invention relates to anthracene derivatives and organic electroluminescent devices made by using the same. More particularly, the present invention relates to anthracene derivatives which enable an organic electroluminescence device to exhibit a great efficiency of light emission and uniform light emission even at high temperatures and organic electroluminescent devices made by using the same.

### BACKGROUND ART

An organic electroluminescence ("electroluminescence" will be occasionally referred to as "EL", hereinafter) device is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W. Tang of Eastman Kodak Company (C. W. Tang and S. A. Vanslyke, Applied Physics Letters, Volume 51, Pages 913, 1987), many studies have been conducted on organic EL devices using organic materials as the constituting materials. Tang et al. used a laminate structure using tris(8-hydroxyquinolinol)aluminum for the light emitting layer and a triphenyldiamine derivative for the hole transporting layer. Advantages of the laminate structure are that the efficiency of hole injection into the light emitting layer can be increased, that the efficiency of forming excited particles which are formed by blocking and recombining electrons injected from the cathode can be increased, and that excited particles formed within the light emitting layer can be enclosed. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

As the light emitting material, chelate complexes such as tris(8-quinolinolato)aluminum, coumarine derivatives, tetraphenylbutadiene derivatives, bisstyrylarylene derivatives and oxadiazole derivatives are known. It is reported that light in the visible region ranging from blue light to red light can be obtained by using these light emitting materials, and development of a device exhibiting color images is expected (For example, Japanese Patent Application Laid-Open Nos. Heisei 8(1996)-239655, Heisei 7(1995)-138561 and Heisei 3(1991)-200289).

For the practical use of an organic EL device, stability of driving for a long period of time, stability of driving under environments of high temperatures such as the environment in automobiles and stability in storage have been required. In relation to these requirements, a great problem is present in that uniformity of light emission by the device is adversely affected by crystallization of the constituting components under the above environments. When a device is driven for a long period of time, the constituting components of the device are exposed to great thermal changes due to the elevation of the temperature by the heat generated by the device itself and due to heat caused by changes in the environment. It is known that organic compounds are crystallized as the result of the exposure to these thermal changes. Since the crystallization causes formation of short circuits and defects, uniformity of the light emitting surface is adversely affected, and occasionally the light emission stops. Therefore, the technology for suppressing crystallization has been studied.

A device using a phenylanthracene derivative as the light emitting material is disclosed in Japanese Patent Application Laid-Open No. Heisei 8(1996)-012600. The above anthracene derivative is used as the material for emitting bluish light, and improvement in the stability of the thin film has been required so that the life of the device can be increased. However, conventional monoanthracene derivatives frequently crystallize to cause destruction of the thin film, and the improvement has been desired. A light emitting device using a compound in which the anthracene ring and the fluorene ring are directly bonded to each other is disclosed in Japanese Patent Application Laid-Open No. 2002-154993. However, the improvement in the uniformity of light emission at high temperatures has not been achieved.

A light emitting material which is a substituted anthracene having spirofluorene at the 9- and 10-positions is disclosed (Japanese Patent Application Laid-Open No. 2002-121547). This light emitting material has a drawback in that, although an improvement is shown with respect to the crystallization, a high temperature of 400°C or higher is necessary as the temperature of vaporization since the skeleton structures of spirofluorene having the great molecular weight are present at the two positions, and the blue light cannot be emitted since thermal decomposition takes place during the vapor deposition.

### DISCLOSURE OF THE INVENTION

The present invention has been made to overcome the above problems and has an object of providing an anthracene derivative which enables an organic EL device to exhibit a great efficiency of light emission and uniform light emission even at high temperatures and an organic EL device utilizing the derivative.

As the result of intensive studies to achieve the above object, it was found that a specific anthracene derivative represented by general formula (1) below suppressed crystallization, had a high glass transition temperature and, when the derivative was used as the light emitting material or the hole transporting material of an organic EL device, provided a great efficiency of light emission and enabled to exhibit uniform light emission even at high temperatures. The present invention has been completed based on this knowledge.

The present invention provides an anthracene derivative represented by following general formula (1): 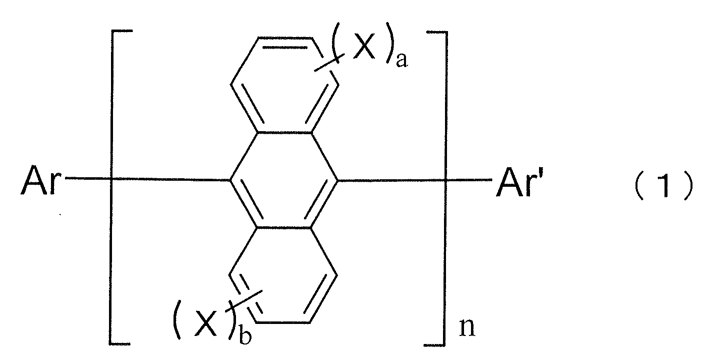 wherein
Ar represents a substituted or unsubstituted group represented by following general formula (2): 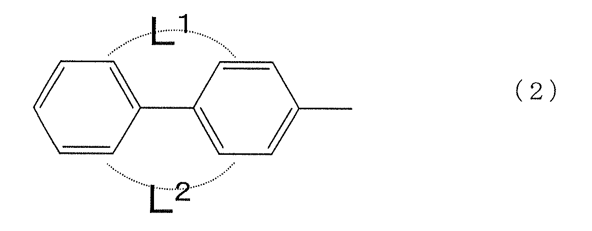 in general formula (2), L¹ and L² each representing a substituted or unsubstituted linking group which forms a cyclic structure, and at least one of the groups represented by L¹ and L² being present,
Ar' represents a substituted or unsubstituted aryl group having 6 to 50 nuclear carbon atoms,
X represents a substituted or unsubstituted alkyl group having 1 to 50 carbon atoms, a substituted or unsubstituted alkoxyl group having 1 to 50 carbon atoms, a substituted or unsubstituted cycloalkyl group having 5 to 50 carbon atoms, a substituted or unsubstituted aralkyl group having 6 to 60 carbon atoms, a substituted or unsubstituted aryl group having 6 to 50 nuclear carbon atoms, a substituted or unsubstituted aromatic heterocyclic group having 5 to 50 nuclear atoms, a substituted or unsubstituted aryloxyl group having 5 to 50 nuclear atoms or a substituted or unsubstituted arylthio group having 5 to 50 nuclear atoms,
   a and b each represent an integer of 0 to 4 and when a plurality of groups represented by X are present, they may be the same with or different from each other, and
   n represents an integer of 1 to 3 and, when n represents 2 or 3, a plurality of groups represented by: 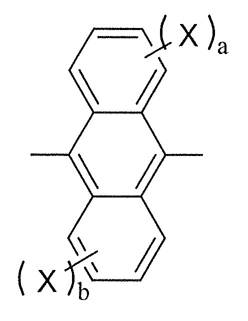
may be a same with or different from each other; with proviso that
when Ar represents a group represented by a following general formula (3): 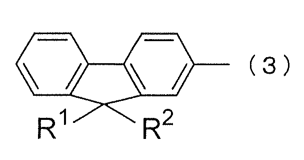 wherein R¹ and R² each represent hydrogen atom, a substituted or unsubstituted alkyl group having 1 to 6 carbon atoms, a substituted or unsubstituted alkoxyl group having 1 to 6 carbon atoms or a substituted or unsubstituted phenyl group,
(i) Ar' represents an aryl group represented by following general formula (4): 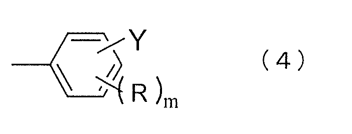 wherein Y represents a substituted or unsubstituted aromatic condensed cyclic residue group having 10 or more nuclear atoms or a substituted or unsubstituted aromatic non-condensed cyclic residue group having 12 or more nuclear atoms, R represents a substituted or unsubstituted alkyl group having 1 to 50 carbon atoms, a substituted or unsubstituted alkoxyl group having 1 to 50 carbon atoms, a substituted or unsubstituted aryl group having 6 to 50 nuclear carbon atoms, a substituted or unsubstituted aromatic heterocyclic group having 5 to 50 nuclear atoms, a substituted or unsubstituted aryloxyl group having 5 to 50 nuclear atoms or a substituted or unsubstituted arylthio group having 5 to 50 nuclear atoms, and m represents an integer of 0 to 4, or
(ii) at least one of a and b does not represent 0, and X represents a substituted or unsubstituted alkyl group having 4 to 50 carbon atoms, a substituted or unsubstituted alkoxyl group having 4 to 50 carbon atoms, a substituted or unsubstituted cycloalkyl group having 5 to 50 carbon atoms, a substituted or unsubstituted aralkyl group having 6 to 60 carbon atoms, a substituted or unsubstituted aryl group having 10 to 50 nuclear carbon atoms, a substituted or unsubstituted aromatic heterocyclic group having 10 to 50 nuclear atoms, a substituted or unsubstituted aryloxyl group having 5 to 50 nuclear atoms or a substituted or unsubstituted arylthio group having 5 to 50 nuclear atoms, and
   when Ar represents a group represented by a following general formula (3'): 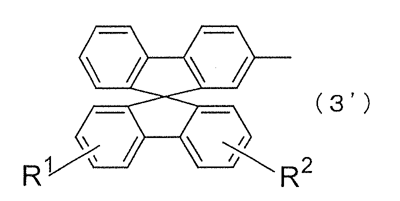
wherein R¹ and R² are as defined above, Ar' represents an aryl group represented by the foregoing general formula (4).

The present invention also provides an organic electroluminescence device which comprises a cathode, an anode and an organic thin film layer comprising at least one layer including a light emitting layer and sandwiched between the cathode and the anode, wherein at least one layer in the organic thin film layer comprises an anthracene derivative represented by general formula (1) described in Claim 1 singly or as a component of a mixture.

### THE MOST PREFERRED EMBODIMENT TO CARRY OUT THE INVENTION

The anthracene derivative of the present invention comprises a compound represented by following general formula (1): 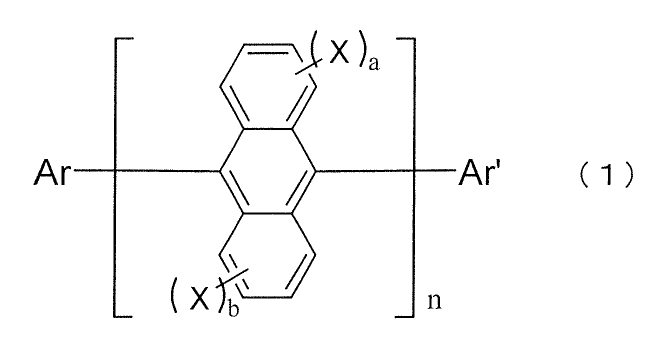 wherein Ar represents a substituted or unsubstituted group represented by following general formula (2): 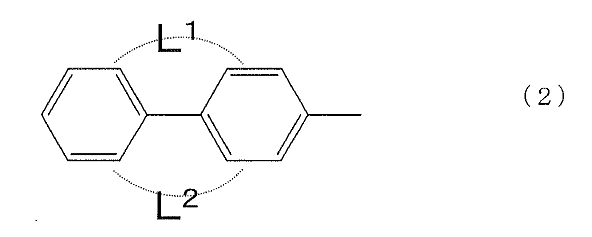 that may be substituted. L¹ and L² each represent a substituted or unsubstituted linking group which forms a cyclic structure and at least one of the groups represented by L¹ and L² is present.

The linking group represented by L¹ and L² is not particularly limited as long as two phenyl groups are linked through one or more carbon atoms and the two phenyl groups are not conjugated. Examples of the linking group include groups having the structure of methylene group, ethylene group, dimethylmethylene group, diphenylmethylene group, lactone ring and peptide group. The linking groups having the structure of methylene group or ethylene group are preferable.

Specific examples of the group represented by Ar are shown in the following. These groups may each have substituents.

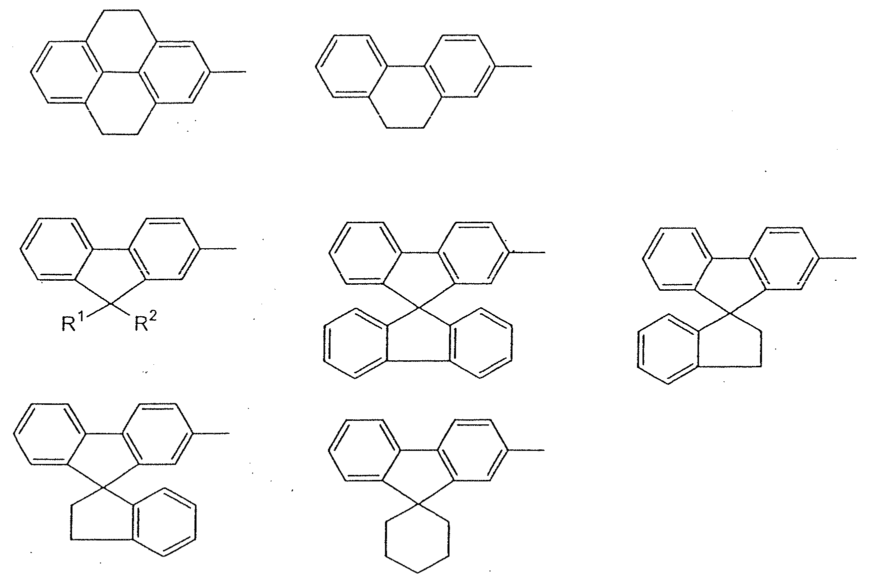

In the above formulae, R¹ and R² are as defined antecedently.

Ar' represents a substituted or unsubstituted aryl group having 6 to 50 nuclear carbon atoms, examples of which include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methyl-biphenylyl group, 4"-t-butyl-p-terphenyl-4-yl group, fluorenyl group and groups represented by Ar described antecedently.

X represents a substituted or unsubstituted alkyl group having 1 to 50 carbon atoms, a substituted or unsubstituted alkoxyl group having 1 to 50 carbon atoms, a substituted or unsubstituted cycloalkyl group having 5 to 50 carbon atoms, a substituted or unsubstituted aralkyl group having 6 to 60 carbon atoms, a substituted or unsubstituted aryl group having 6 to 50 nuclear carbon atoms, a substituted or unsubstituted aromatic heterocyclic group having 5 to 50 nuclear atoms, a substituted or unsubstituted aryloxyl group having 5 to 50 nuclear atoms or a substituted or unsubstituted arylthio group having 5 to 50 nuclear atoms.

Examples of the substituted or unsubstituted alkyl group represented by X include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3- tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3- tricyano- propyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group and 1,2,3-trinitropropyl group.

The substituted or unsubstituted alkoxyl group represented by X is a group represented by -OA. Examples of the group represented by A include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxy- isopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3- tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group and 1,2,3-trinitropropyl group.

Examples of the substituted or unsubstituted cycloalkyl group represented by X include cyclopropyl group, cyclobutyl group, cyclopentyl group, cyclohexyl group, 4-methylcyclohexyl group, adamantyl group and norbornyl group.

Examples of the substituted or unsubstituted aralkyl group represented by X include benzyl group, 1-phenylethyl group, 2-phenylethyl group, 1-phenyl- isopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group, 1-chloro-2-phenylisopropyl group and trityl group.

Examples of the substituted or unsubstituted aryl group represented by X include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group and 4"-t-butyl-p-terphenyl-4-yl group.

Examples of the substituted or unsubstituted aromatic heterocyclic group represented by X include 1-pyrrolyl group, 2-pyrrolyl group, 3-pyrrolyl group, pyradinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-carbazolyl group, 2-carbazolyl group, 3-carbazolyl group, 4-carbazolyl group, 9-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group and 4-t-butyl-3-indolyl group.

The substituted or unsubstituted aryloxyl group represented by X is a group represented by -OZ. Examples of the group represented by Z include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl- p-terphenyl-4-yl group, 2-pyrrolyl group, 3-pyrrolyl group, pyradinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 1-phenoxazinyl group, 2-phenoxazinyl group, 3-phenoxazinyl group, 4-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl- pyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group and 4-t-butyl-3-indolyl group.

The substituted or unsubstituted arylthio group represented by X is a group represented by -SZ. Examples of the group represented by Z include the same groups described as the examples of the group represented by Z in the aryloxyl group.

In general formula (1), a and b each represent an integer of 0 to 4 and preferably 0 or 1. When a plurality of groups represented by X are present, the plurality of groups may be the same with or different from each other.
n represents an integer of 1 to 3. When n represents 2 or 3, a plurality of groups represented by: 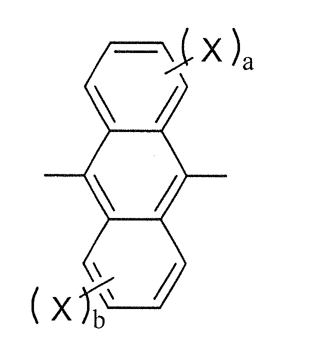 may be the same with or different from each other.

In general, the anthracene derivative exhibits great crystallinity, and there is the anxiety that the uniformity of light emission and the yield of the device decrease when the anthracene derivative is used as the light emitting material of organic EL devices.

Therefore, in the anthracene derivative of the present invention, when Ar in general formula (1) represents a group represented by the following general formula (3): 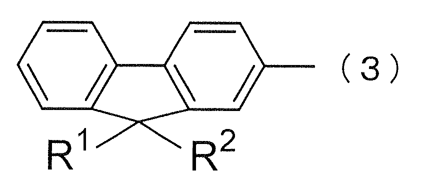
(i) Ar' represents an aryl group represented by the following general formula (4): 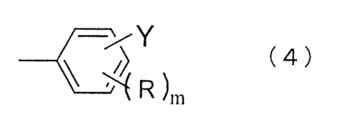 or
(ii) at least one of a and b does not represent 0, and X represents a substituted or unsubstituted alkyl group having 4 to 50 carbon atoms, a substituted or unsubstituted alkoxyl group having 4 to 50 carbon atoms, a substituted or unsubstituted cycloalkyl group having 5 to 50 carbon atoms, a substituted or unsubstituted aralkyl group having 6 to 60 carbon atoms, a substituted or unsubstituted aryl group having 10 to 50 nuclear carbon atoms, a substituted or unsubstituted aromatic heterocyclic group having 10 to 50 nuclear atoms, a substituted or unsubstituted aryloxyl group having 5 to 50 nuclear atoms or a substituted or unsubstituted arylthio group having 5 to 50 nuclear atoms.

When Ar represents a group represented by the following general formula (3'): 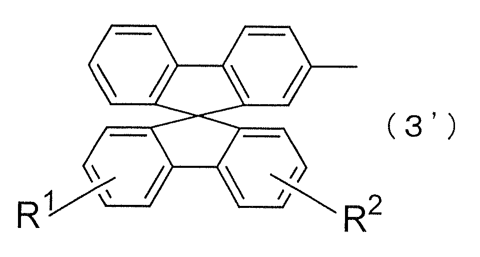 Ar' represents an aryl group represented by the above general formula (4).

In general formulae (3) and (3'), R¹ and R² each represent hydrogen atom, a substituted or unsubstituted alkyl group having 1 to 6 carbon atoms, a substituted or unsubstituted alkoxyl group having 1 to 6 carbon atoms or a substituted or unsubstituted phenyl group.

In general formula (4), Y represents a substituted or unsubstituted aromatic condensed cyclic residue group having 10 or more nuclear atoms or a substituted or unsubstituted aromatic non-condensed cyclic residue group having 12 or more nuclear atoms.

Examples of the aromatic condensed cyclic compound from which the group represented by Y derives include naphthalene, fluoranthene, perylene, pentacene, phenanthrene, chrysene, benzanthracene and pyrene.

Examples of the aromatic non-condensed cyclic compound from which the group represented by Y derives include biphenyl, terphenyl, and quarterohenyl.

R represents a substituted or unsubstituted alkyl group having 1 to 50 carbon atoms, a substituted or unsubstituted alkoxyl group having 1 to 50 carbon atoms, a substituted or unsubstituted aryl group having 6 to 50 nuclear carbon atoms, a substituted or unsubstituted aromatic heterocyclic group having 5 to 50 nuclear atoms, a substituted or unsubstituted aryloxyl group having 5 to 50 nuclear atoms or a substituted or unsubstituted arylthio group having 5 to 50 nuclear atoms. Examples of the above groups include the same groups as those described antecedently as the examples of the group represented by X.

m represents an integer of 0 to 4.

Examples of the substituent to the groups represented by X, Ar, Ar', R¹, R², Y and R include halogen atoms, hydroxyl group, nitro group, cyano group, alkyl groups, aryl groups, cycloalkyl groups, alkoxyl groups, aromatic heterocyclic groups, aralkyl groups, aryloxyl groups, arylthio groups, alkoxycarbonyl groups and carboxyl group.

Examples of the anthracene derivative represented by general formula (1) are shown in, but not limited to, the compounds shown as the following:

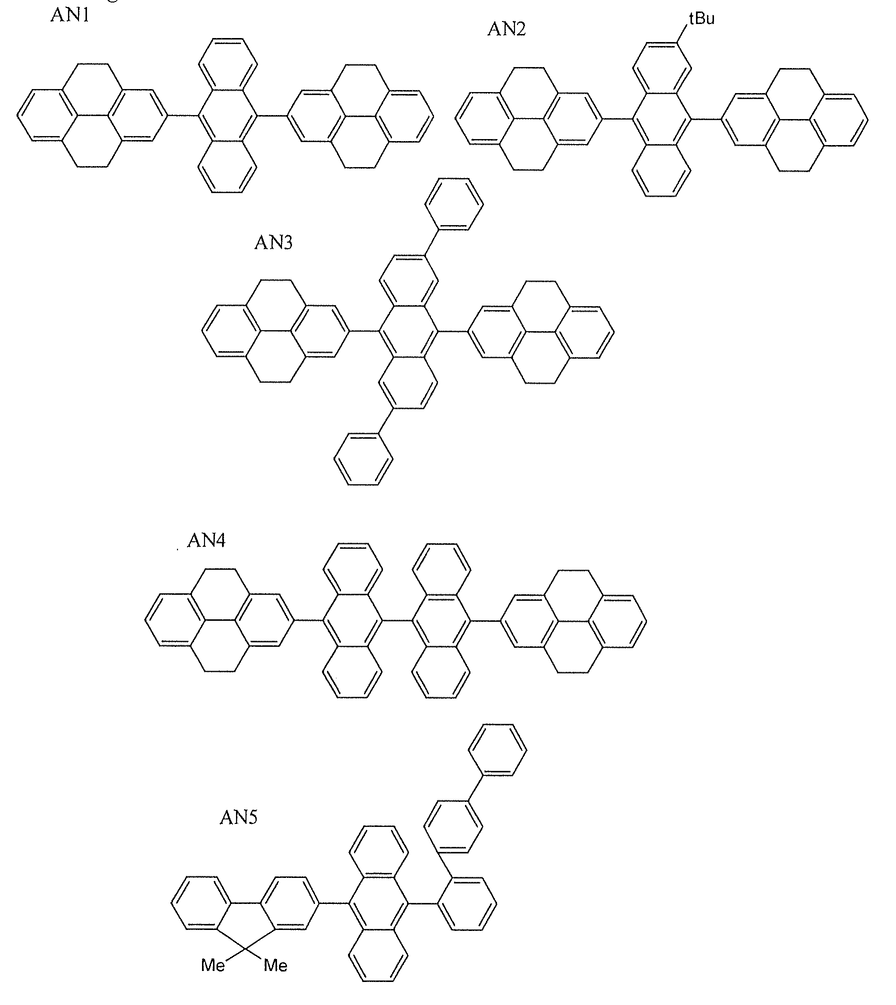

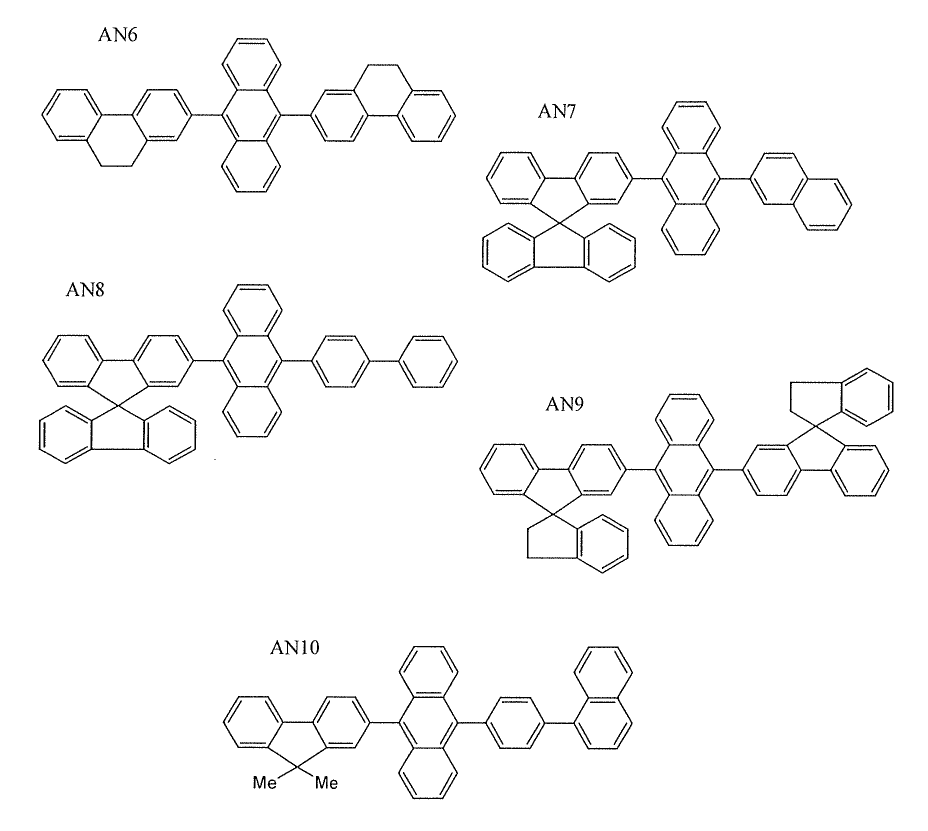

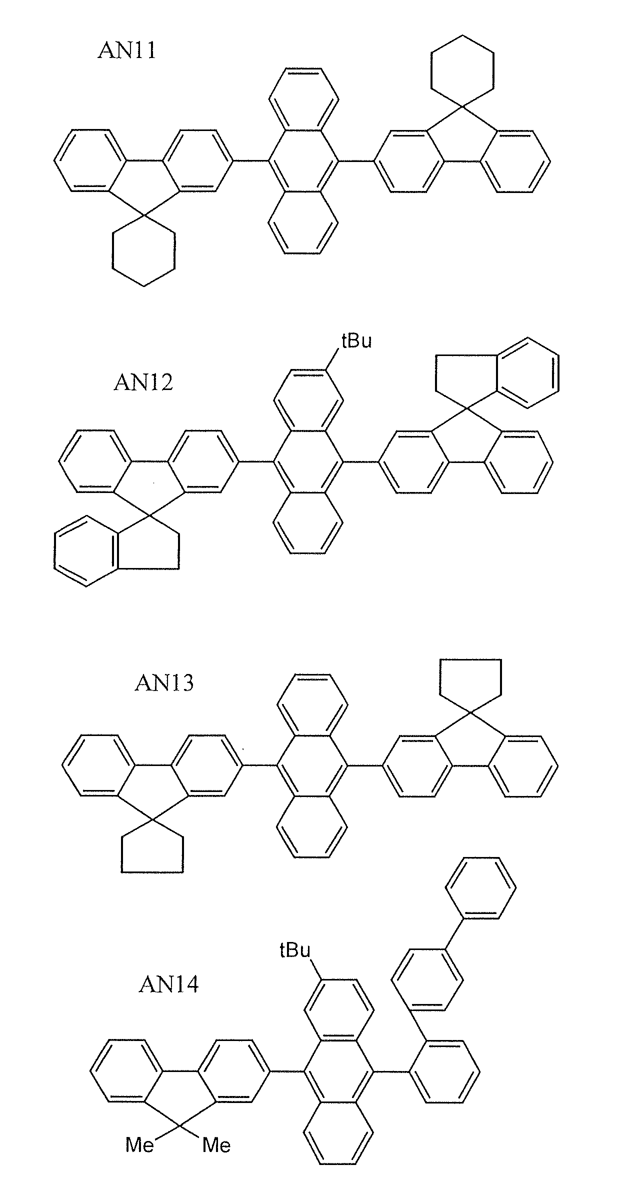

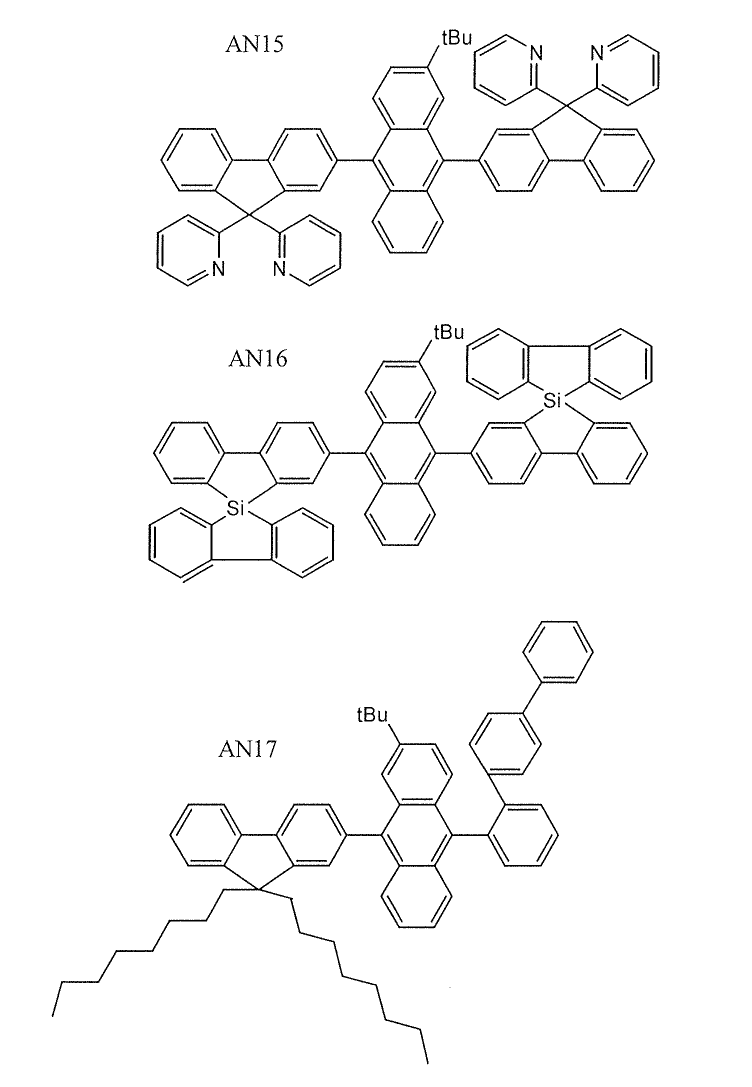

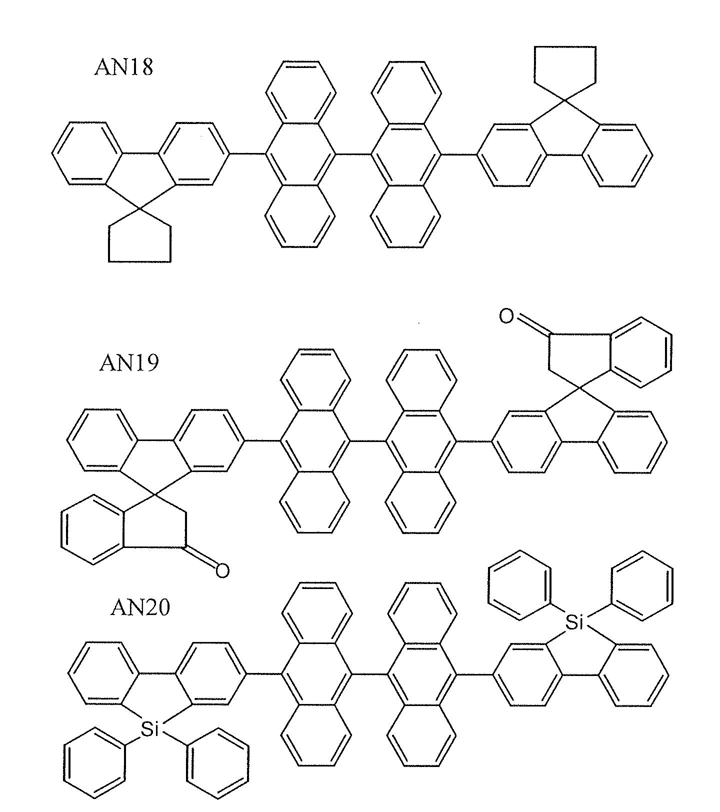

It is preferable that the anthracene derivative of the present invention is used as the light emitting material or the hole transporting material of organic EL devices.

The organic EL device of the present invention comprises a cathode, an anode and an organic thin film layer comprising at least one layer including a light emitting layer and sandwiched between the cathode and the anode, wherein at least one layer in the organic thin film layers comprises the anthracene derivative represented by general formula (1) described above singly or as a component of a mixture.

It is preferable that the light emitting layer comprises the anthracene derivative represented by general formula (1). It is more preferable that the light emitting layer comprises the anthracene derivative represented by general formula (1) as the main component.

It is preferable that the light emitting layer in the organic EL device of the present invention further comprises an arylamine compound and/or a styrylamine compound.

As the styrylamine compound, compounds represented by the following general formula (A): 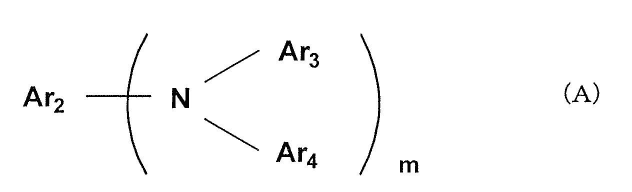 wherein Ar₂ represent a group selected from phenyl group, biphenyl group, terphenyl group, stilbene group and distyrylaryl groups, Ar₃ and Ar₄ each represent hydrogen atom or an aromatic group having 6 to 20 carbon atoms, the groups represented by Ar₂, Ar₃ and Ar₄ may be substituted, m represents an integer of 1 to 4 and, preferably, at least one of the groups represented by Ar₃ and Ar₄ is substituted with styryl group, are preferable.

Examples of the aromatic group having 6 to 20 carbon atoms include phenyl group, naphthyl group, anthranyl group, phenanthryl group and terphenyl group.

As the arylamine compound, compounds represented by the following general formula (B): 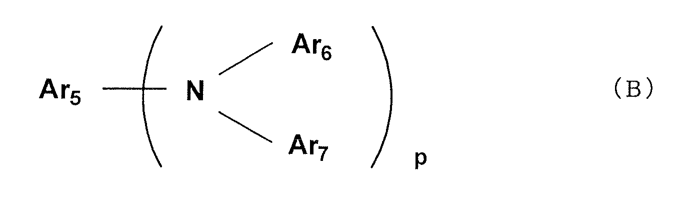 wherein Ar₅ to Ar₇ each represent an aryl group having 5 to 40 nuclear carbon atoms, and p represents an integer of 1 to 4, are preferable.

Examples of the aryl group having 5 to 40 nuclear carbon atoms include phenyl group, naphthyl group, anthranyl group, phenanthryl group, pyrenyl group, coronyl group, biphenyl group, terphenyl group, pyrrolyl group, furanyl group, thiophenyl group, benzothiophenyl group, oxadiazolyl group, diphenylanthranyl group, indolyl group, carbazolyl group, pyridyl group, benzoquinolyl group, fluoranthenyl group, acenaphthofluoranthenyl group and stilbene. Preferable examples of the substituent to the aryl group include alkyl groups having 1 to 6 carbon atoms such as ethyl group, methyl group, 1-propyl group, n-propyl group, s-butyl group, t-butyl group, pentyl group, hexyl group, cyclopentyl group and cyclohexyl group; alkoxyl groups having 1 to 6 carbon atoms such as ethoxyl group, methoxyl group, i-propoxyl group, n-propoxyl group, s-butoxyl group, t-butoxyl group, pentoxyl group, hexyloxyl group, cyclopentoxyl group and cyclohexyloxyl group; aryl groups having 5 to 40 nuclear atoms; amino groups substituted with an aryl group having 5 to 40 nuclear atoms; ester groups having an aryl group having 5 to 40 nuclear atoms; ester groups having an alkyl group having 1 to 6 carbon atoms; cyano group; nitro group; and halogen atoms.

The organic thin film layers may include a hole transporting layer, and the hole transporting layer may comprise the anthracene derivative represented by general formula (1) singly or as a component of a mixture. It is preferable that the hole transporting layer comprises the anthracene derivative as the main component.

The construction of the device in the organic EL device of the present invention will be explained below.

Typical examples of the construction of the organic EL device include:
(1) An anode / a light emitting layer / a cathode;
(2) An anode / a hole injecting layer / a light emitting layer / a cathode;
(3) An anode / a light emitting layer / an electron injecting layer / a cathode;
(4) An anode / a hole injecting layer / a light emitting layer / an electron injecting layer / a cathode;
(5) An anode / an organic semiconductor layer / a light emitting layer / a cathode;
(6) An anode / an organic semiconductor layer / an electron barrier layer / a light emitting layer / a cathode;
(7) An anode / an organic semiconductor layer / a light emitting layer / an adhesion improving layer / a cathode;
(8) An anode / a hole injecting layer / a hole transporting layer / a light emitting layer / an electron injecting layer / a cathode;
(9) An anode / an insulating layer / a light emitting layer / an insulating layer / a cathode;
(10) An anode / an inorganic semiconductor layer / an insulating layer / a light emitting layer / an insulating layer / a cathode;
(11) An anode / an organic semiconductor layer / an insulating layer / a light emitting layer / an insulating layer / a cathode;
(12) An anode / an insulating layer / a hole injecting layer / a hole transporting layer / a light emitting layer / an insulating layer / a cathode; and
(13) An anode / an insulating layer / a hole injecting layer / a hole transporting layer / a light emitting layer / an electron injecting layer / a cathode.

Among the above constructions, construction (8) is preferable. However, the construction of the organic EL device is not limited to those shown above as the examples.

In general, the organic EL device is produced on a substrate which transmits light. The substrate which transmits light is the substrate which supports the organic EL device. It is preferable that the substrate which transmits light has a transmittance of light of 50% or greater in the visible region of 400 to 700 nm. It is also preferable that a flat and smooth substrate is employed.

As the substrate which transmits light, for example, glass sheet and synthetic resin sheet are advantageously employed. Specific examples of the glass sheet include soda ash glass, glass containing barium and strontium, lead glass, aluminosilicate glass, borosilicate glass, barium borosilicate glass and quartz. Specific examples of the synthetic resin sheet include sheet made of polycarbonate resins, acrylic resins, polyethylene terephthalate resins, polyether sulfide resins and polysulfone resins.

As the anode, an electrode made of a material such as a metal, an alloy, a conductive compound and a mixture of these materials which has a great work function (4 eV or more) is preferable. Specific examples of the material for the anode include metals such as Au and conductive materials such as CuI, ITO (indium tin oxide), SnO₂ ZnO and In-Zn-O. The anode can be prepared by forming a thin film of the electrode material described above in accordance with a process such as the vapor deposition process and the sputtering process. When the light emitted from the light emitting layer is obtained through the anode, it is preferable that the anode has a transmittance of the emitted light greater than 10%. It is also preferable that the sheet resistivity of the anode is several hundred Ω/□ or smaller. The thickness of the anode is, in general, selected in the range of from 10 nm to 1 µm and preferably in the range of from 10 to 200 nm although the preferable range may be different depending on the adopted material.

As the cathode, an electrode made of a material such as a metal, an alloy, a conductive compound and a mixture of these materials which has a small work function (4 eV or smaller) is employed. Specific examples of the material for the cathode include sodium, sodium-potassium alloys, magnesium, lithium, magnesium-silver alloys, aluminum/aluminum oxide, Al/Li₂O, Al/LiO₂, Al/LiF, aluminum-lithium alloys, indium and rare earth metals.

The cathode can be prepared by forming a thin film of the electrode material described above in accordance with a process such as the vapor deposition process and the sputtering process.

When the light emitted from the light emitting layer is obtained through the cathode, it is preferable that the cathode has a transmittance of the emitted light greater than 10 %. It is also preferable that the sheet resistivity of the cathode is several hundred Ω/□ or smaller. The thickness of the cathode is, in general, selected in the range of from 10 nm to 1 µm and preferably in the range of from 50 to 200 nm.

In the organic EL device of the present invention, it is preferable that a layer of a chalcogenide, a metal halide or a metal oxide (this layer may occasionally be referred to as a surface layer) is disposed on the surface of at least one of the pair of electrodes prepared as described above. Specifically, it is preferable that a layer of a chalcogenide (including an oxide) of a metal such as silicon and aluminum is disposed on the surface of the anode at the side of the light emitting layer, and a layer of a metal halide or a metal oxide is disposed on the surface of the cathode at the side of the light emitting layer. Due to the above layers, stability in driving can be improved.

Preferable examples of the chalcogenide include SiOₓ (1 ≦ x ≦ 2), AlOₓ (1 ≦ x ≦ 1.5), SiON and SiAlON. Preferable examples of the metal halide include LiF, MgF₂, CaF₂ and fluorides of rare earth metals. Preferable examples of the metal oxide include Cs₂O, Li₂O, MgO, SrO, BaO and CaO.

In the organic EL device of the present invention, it is preferable that a mixed region of an electron transfer compound and a reducing dopant or a mixed region of a hole transfer compound and an oxidizing dopant is disposed on the surface of at least one of the pair of electrodes prepared as described above. Due to the mixed region disposed as described above, the electron transfer compound is reduced to form an anion, and injection and transportation of electrons from the mixed region into the light emitting medium can be facilitated. The hole transfer compound is oxidized to form a cation, and injection and transportation of holes from the mixed region into the light emitting medium is facilitated. Preferable examples of the oxidizing dopant include various types of Lewis acid and acceptor compounds. Preferable examples of the reducing dopant include alkali metals, compounds of alkali metals, alkaline earth metals, rare earth metals and compounds of these metals.

In the organic EL device of the present invention, the light emitting layer has the following functions:
(1) The injecting function: the function of injecting holes from the anode or the hole injecting layer and injecting electrons from the cathode or the electron injecting layer when an electric field is applied;
(2) The transporting function: the function of transporting injected charges (electrons and holes) by the force of the electric field; and
(3) The light emitting function: the function of providing the field for recombination of electrons and holes and leading the recombination to the emission of light.

As the process for forming the light emitting layer, a well known process such as the vapor deposition process, the spin coating process and the LB process can be employed. It is particularly preferable that the light emitting layer is a molecular deposit film. The molecular deposit film is a thin film formed by deposition of a material compound in the gas phase or a thin film formed by solidification of a material compound in a solution or in the liquid phase. In general, the molecular deposit film can be distinguished from the thin film formed in accordance with the LB process (the molecular accumulation film) based on the differences in the aggregation structure and higher order structures and functional differences caused by these structural differences.

As disclosed in Japanese Patent Application Laid-Open No. Showa 57(1982)-51781, the light emitting layer can also be formed by dissolving a binder such as a resin and the material compounds into a solvent to prepare a solution, followed by forming a thin film from the prepared solution in accordance with the spin coating process or the like.

In the present invention, where desired, the light emitting layer may comprise well known light emitting materials other than the light emitting material comprising the anthracene derivative of the present invention, or a light emitting layer comprising other well known light emitting material may be laminated to the light emitting layer comprising the light emitting material comprising the anthracene derivative of the present invention as long as the object of the present invention is not adversely affected.

The hole injecting layer and the hole transporting layer are layers which help injection of holes into the light emitting layer and transport the holes to the light emitting region. The layers exhibit a great mobility of holes and, in general, have an ionization energy as small as 5.5 eV or smaller. For the hole injecting layer and the hole transporting layer, a material which transports holes to the light emitting layer at a small strength of the electric field is preferable. A material which exhibits, for example, a mobility of holes of at least 10⁻⁶ cm²/V·sec under application of an electric field of from 10⁴ to 10⁶ V/cm is preferable. The anthracene derivative of the present invention is useful as the hole transporting material. A material other than the anthracene derivative can be selected from materials which are conventionally employed as the charge transporting material of holes in photoconductive materials and well known materials which are employed for the hole injecting layer in organic EL devices.

To form the hole injecting layer or the hole transporting layer, a thin film may be formed from the material for the hole injecting layer or the hole transporting layer, respectively, in accordance with a well known process such as the vacuum vapor deposition process, the spin coating process, the casting process and the LB process. Although the thickness of the hole injecting layer and the hole transporting layer is not particularly limited, the thickness is usually from 5 nm to 5 µm.

The electron injection layer and the electron transporting are layers which help injection of electrons into the light emitting layer and transports electrons to the light emitting region and exhibit a great mobility of electrons. Among the electron injecting layers, an adhesion improving layer is a layer made of a material exhibiting excellent adhesion with the cathode. As the material for the electron injecting layer, metal complexes of 8-hydroxyquinoline and derivatives thereof are preferable. Examples of the metal complex of 8-hydroxyquinoline and derivatives thereof include metal chelates of oxinoid compounds including chelates of oxine (in general, 8-quinolinol or 8-hydroxyquinoline). For example, tris(8-quinolinol)aluminum can be employed as the electron injecting material.

In general, an organic EL device tends to form defects in pixels due to leak and short circuit since an electric field is applied to ultra-thin films. To prevent the formation of the defects, a layer of an insulating thin film may be inserted between the pair of electrodes.

Examples of the material employed for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide. Mixtures and laminates of the above compounds can also be employed.

To produce the organic EL device of the present invention, for example, the anode, the light emitting layer and, where necessary, the hole injecting layer and the electron injecting layer are formed in accordance with the above process using the above materials, and the cathode is formed in the last step. The organic EL device may be produced by forming the above layers in the order reverse to that described above, i.e., the cathode being formed in the first step and the anode in the last step.

An embodiment of the process for producing an organic EL device having a construction in which an anode, a hole injecting layer, a light emitting layer, an electron injecting layer and a cathode are disposed successively on a substrate transmitting light will be described in the following.

On a suitable substrate transmitting light, a thin film made of a material for the anode is formed in accordance with the vapor deposition process or the sputtering process so that the thickness of the formed thin film is 1 µm or smaller and preferably in the range of 10 to 200 nm. The formed thin film is employed as the anode. Then, a hole injecting layer is formed on the anode. The hole injecting layer can be formed in accordance with the vacuum vapor deposition process, the spin coating process, the casting process or the LB process, as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions are suitably selected in the following ranges: the temperature of the source of the deposition: 50 to 450°C; the vacuum: 10⁻⁷ to 10⁻³ Torr; the rate of deposition: 0.01 to 50 nm/second; the temperature of the substrate: -50 to 300°C and the thickness of the film: 5 nm to 5 µm; although the conditions of the vacuum vapor deposition are different depending on the employed compound (the material for the hole injecting layer) and the crystal structure and the recombination structure of the hole injecting layer to be formed.

Then, the light emitting layer is formed on the hole injecting layer formed above. Using the light emitting material described in the present invention, a thin film of the light emitting material can be formed in accordance with the vacuum vapor deposition process, the sputtering process, the spin coating process or the casting process, and the formed thin film is employed as the light emitting layer. The vacuum vapor deposition process is preferable because a uniform film can be easily obtained and the possibility of formation of pin holes is small. When the light emitting layer is formed in accordance with the vacuum vapor deposition process, in general, the conditions of the vacuum vapor deposition process can be selected in the same ranges as those described for the vacuum vapor deposition of the hole injecting layer although the conditions are different depending on the used compound. It is preferable that the thickness is in the range of from 10 to 40 nm.

An electron injecting layer is formed on the light emitting layer formed above. Similarly to the hole injecting layer and the light emitting layer, it is preferable that the electron injecting layer is formed in accordance with the vacuum vapor deposition process since a uniform film must be obtained. The conditions of the vacuum vapor deposition can be selected in the same ranges as those described for the vacuum vapor deposition of the hole injecting layer and the light emitting layer.

A cathode is formed on the electron injecting layer formed above in the last step, and an organic EL device can be obtained. The cathode is made of a metal and can be formed in accordance with the vacuum vapor deposition process or the sputtering process. It is preferable that the vacuum vapor deposition process is employed in order to prevent appearance of damages on the lower organic layers during the formation of the film.

In the above production of the organic EL device, it is preferable that the above layers from the anode to the cathode are formed successively while the production system is kept in a vacuum after being evacuated.

The organic EL device which can be produced as described above emits light when a direct voltage of 3 to 40 V is applied in the condition that the anode is connected to a positive electrode (+) and the cathode is connected to a negative electrode (-). When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be employed.

The present invention will be described more specifically with reference to examples in the following. However, the present invention is not limited to the examples.

### Example 1 (Synthesis of a compound AN1)

### (1) Synthesis of 4,5,9,10-tetrahydro-2-bromopyrene

Into an autoclave, 195 g of pyrene (available from HIROSHIMA WAKO Co., Ltd.), 1 liter of decaline (available from HIROSHIMA WAKO Co., Ltd.) and 78 g of 5% palladium carbon (available from HIROSHIMA WAKO Co., Ltd.) were placed, and the reaction was allowed to proceed at 160°C for 21 hours under a hydrogen pressure of 70 kg/cm².

After the reaction was completed, the catalyst was separated by filtration and washed with 3 liters of chloroform. Then, chloroform was removed under a reduced pressured, and the remaining decaline solution was cooled with ice. The formed crystals were separated by filtration, washed with ethanol and dried, thereby obtaining 130 g of crystals.

The obtained crystals in an amount of 126 g was suspended in 6.3 liters of purified water, and 2 g of ferric chloride monohydrate (available from HIROSHIMA WAKO Co., Ltd.) was added to the suspension. Then, an aqueous solution obtained from 30 milliliter of bromine and 3 liters of purified water was added dropwise at the room temperature over 4 hours. The reaction was then allowed to proceed at the room temperature for 12 hours.

The formed crystals were separated by filtration, washed with water and ethanol and dissolved into 3 liters of chloroform. The resultant solution was washed with an aqueous solution of sodium hydrogencarbonate and water and dried with anhydrous magnesium sulfate, and the solvent was then removed.

To the obtained residue, 1.5 liters of hexane was added. The formed crystals were separated by filtration, and 71.5 g of the crystals were obtained.

Since m/z=286 and 284 in the field desorption mass analysis (FD-MS) of the obtained compound, which corresponded to C₁₀H₁₂Br=285, the compound was identified to be 4,5,9,10-tetrahydro-2-bromopyrene (the yield: 41%).

### (2) Synthesis of a compound AN1

Under the atmosphere of argon, 2 g of 4,5,9,10-tetrahydro-2-bromopyrene obtained in (1) described above was dissolved into a mixed solvent of 8 milliliter of anhydrous tetrahydrofuran (THF) and 8 milliliter of anhydrous toluene, and the resultant solution was cooled at -20°C in a dry ice/methanol bath. To the cooled solution, 5 milliliter of a hexane solution of n-butyllithium (1.6 moles/liter; available from HIROSHIMA WAKO Co., Ltd.) was added, and the resultant solution was stirred at -20°C for 1 hour. Then, 0.62 g of 9,10-anthraquinone (available from TOKYO KASEI Co., Ltd.) was added, and the resultant solution was stirred at the room temperature for 4 hours and left standing at the room temperature for 12 hours.

The reaction mixture was deactivated with a saturated aqueous solution of ammonium chloride, and the formed solid substance was separated by filtration and washed with methanol. The obtained compound was purified in accordance with the column chromatography, and 1.6 g of a light yellow solid substance was obtained.

Since m/z=586 in FD-MS of the obtained compound, which corresponded to C₄₆H₃₄=586, the compound was identified to be AN1 (the yield: 94%).

### Example 2 (Synthesis of a compound AN2)

Under the atmosphere of argon, 2 g of 4,5,9,10-tetrahydro-2-bromopyrene obtained in (1) of Example 1 described above was dissolved into a mixed solvent of 8 milliliter of anhydrous THF and 8 milliliter of anhydrous toluene, and the resultant solution was cooled at -20°C in a dry ice/methanol bath. To the cooled solution, 5 milliliter of a hexane solution of n-butyllithium (1.6 moles/liter; available from HIROSHIMA WAKO Co., Ltd.) was added, and the resultant solution was stirred at -20°C for 1 hour. Then, 0.8 g of 2-t-butylanthraquinone (available from TOKYO KASEI Co., Ltd.) was added, and the resultant solution was stirred at the room temperature for 4 hours and left standing at the room temperature for 12 hours.

The reaction mixture was deactivated with a saturated aqueous solution of ammonium chloride, and the formed solid substance was separated by filtration and washed with methanol. The obtained compound was purified in accordance with the column chromatography, and 1.8 g of a light yellow solid substance was obtained.

Since m/z=642 in FD-MS of the obtained compound, which corresponded to C₅₀H₄₂=642, the compound was identified to be AN2 (the yield: 91%).

### Example 3 (Synthesis of a compound AN3)

### (1) Synthesis of 2,6-diphenyl-9,10-anthraquinone

Into a 3 liter flask, 130 g of 4-bromophthalic anhydride (available from TOKYO KASEI Co., Ltd.), 243 g of sodium carbonate and 1.3 liters of water were placed, and a solution was prepared by heating up to 60°C. After the prepared solution was cooled to the room temperature, 84.5 g of phenylboric acid (available from TOKYO KASEI Co., Ltd.) and 3.9 g of palladium acetate (available from TOKYO KASEI Co., Ltd.) were added, and the resultant mixture was stirred. Then, the reaction was allowed to proceed at the room temperature for 12 hours.

After the reaction was completed, the formed crystals were dissolved by adding water and heating. The catalyst was removed by filtration, and crystals were formed by adding concentrated hydrochloric acid. The formed crystals were separated by filtration and washed with water. After extraction with ethyl acetate, the extract was dried with anhydrous magnesium sulfate and concentrated to remove the entire volatile components, and 145 g of a solid substance was obtained.

The obtained solid substance was placed into 500 milliliter of acetic anhydride (available from HIROSHIMA WAKO Co., Ltd.), and the reaction was allowed to proceed at 80°C for 3 hours. Acetic anhydride was removed under a reduced pressure until the entire volatile components were removed, and 135 g of an acid anhydride was obtained.

Into 670 milliliter of 1,2-dichloroethane, 85.3 g of biphenyl (available from HIROSHIMA WAKO Co., Ltd.) was dissolved. To the resultant solution, 162.7 g of anhydrous aluminum chloride was added, and the obtained mixture was cooled to some degree.

To the obtained mixture, 124 g of the acid anhydride obtained above was added carefully so that excessive heat generation was prevented. After the reaction was allowed to proceed at 40°C for 2 hours, the reaction mixture was poured into ice water, treated by extraction with chloroform, washed with water, dried with anhydrous magnesium sulfate and concentrated. To the obtained mixture, hexane was added, and formed precipitates were separated by filtration.

Polyphosphoric acid in an amount of 2 liters was heated at 150°C. The precipitates separated above were added to the heated polyphosphoric acid in small portions under stirring, and the resultant mixture was stirred at the same temperature for 3 hours.

The reaction mixture was poured into ice water. The formed crystals were separated by filtration, washed with water, dissolved into chloroform, dried with anhydrous magnesium sulfate and purified by a column.

After the object fraction was concentrated, hexane was added, and 98.7 g of the formed crystals were separated by filtration.

Since m/z=360 in the field desorption mass analysis (FD-MS) of the obtained compound, which corresponded to C₂₆H₁₆O₂=360, the compound was identified to be 2,6-diphenyl-9,10-anthraquinone (the yield: 48%).

### (2) Synthesis of a compound AN3

Under the atmosphere of argon, 2 g of 4,5,9,10-tetrahydro-2-bromopyrene obtained in (1) of Example 1 described above was dissolved into a mixed solvent of 8 milliliter of anhydrous THF and 8 milliliter of anhydrous toluene, and the resultant solution was cooled at -20°C in a dry ice/methanol bath. To the cooled solution, 5 milliliter of a hexane solution of n-butyllithium (1.6 moles/liter; available from HIROSHIMA WAKO Co., Ltd.) was added, and the resultant solution was stirred at -20°C for 1 hour. Then, 1.1 g of 2,6-diphenyl-9,10-anthraquinone obtained in (1) described above was added, and the resultant solution was stirred at the room temperature for 4 hours and left standing at the room temperature for 12 hours.

The reaction mixture was deactivated with a saturated aqueous solution of ammonium chloride, and the formed solid substance was separated by filtration and washed with methanol. The obtained compound was purified in accordance with the column chromatography, and 2.0 g of a light yellow solid substance was obtained.

Since m/z=738 in FD-MS of the obtained compound, which corresponded to C₅₈H₄₂=738, the compound was identified to be AN3 (the yield: 89%).

### Example 4 (Synthesis of a compound AN4)

Under the atmosphere of argon, 2 g of 4,5,9,10-tetrahydro-2-bromopyrene obtained in (1) of Example 1 described above was dissolved into a mixed solvent of 8 milliliter of anhydrous THF and 8 milliliter of anhydrous toluene, and the resultant solution was cooled at -20°C in a dry ice/methanol bath. To the cooled solution, 5 milliliter of a hexane solution of n-butyllithium (1.6 moles/liter; available from HIROSHIMA WAKO Co., Ltd.) was added, and the resultant solution was stirred at -20°C for 1 hour. Then, 1.2 g of bianthrone was added, and the resultant solution was stirred at the room temperature for 4 hours and left standing at the room temperature for 12 hours.

The reaction mixture was deactivated with a saturated aqueous solution of ammonium chloride, and the formed solid substance was separated by filtration and washed with methanol. The obtained compound was purified in accordance with the column chromatography, and 2.2 g of a light yellow solid substance was obtained.

Since m/z=763 in FD-MS of the obtained compound, which corresponded to C₆₀H₄₂=762, the compound was identified to be AN4 (the yield: 92%).

### Example 5 (Synthesis of a compound AN5)

### (1) Synthesis of 9,9'-dimethyl-2-bromofluorene

Under the atmosphere of argon, 28 g of 35% potassium hydride (available from HIROSHIMA WAKO Co., Ltd.) was added to 300 milliliter of anhydrous THF, and then 16 g of fluorenone was added. Thereafter, 20 g of iodomethane (available from HIROSHIMA WAKO Co., Ltd.) was added, and the reaction was allowed to proceed at the refluxing temperature for 72 hours.

To the obtained reaction mixture, water was added, and then dilute hydrochloric acid was added. The resultant mixture was treated by extraction with chloroform, and the obtained extract was dried with anhydrous magnesium sulfate. The solvent was removed under a reduced pressure, and the formed solid substance was separated by filtration and washed with methanol.

The solid substance obtained above in an amount of 5 g was suspended into 300 milliliter of purified water, and 0.1 g of ferric chloride monohydrate (available from HIROSHIMA WAKO Co., Ltd.) was added to the resultant suspension. Then, an aqueous solution obtained from 1 milliliter of bromine and 100 milliliter of purified water was added dropwise at the room temperature over 1 hour, and the reaction was allowed to proceed at the room temperature for 12 hours.

After the formed crystals were separated by filtration, washed with water and methanol and dissolved into 200 milliliter of chloroform, the resultant solution was washed with an aqueous solution of sodium hydrogencarbonate and water and dried with anhydrous magnesium sulfate, and the solvent was removed by distillation.

After 100 milliliter of hexane was added to the resultant mixture, the formed crystals were separated by filtration, and 5.4 g of the crystals were obtained.

Since m/z=277 and 275 in FD-MS of the obtained compound, which corresponded to C₁₅H₁₆Br=276, the compound was identified to be 9,9'-dimethyl-2-bromofluorene (the yield: 20%).

### (2) Synthesis of a compound AN5

Under the atmosphere of argon, 1.9 g of 9.9'-dimethyl-2-bromofluorene obtained in (1) described above was dissolved into a mixed solvent of 8 milliliter of anhydrous THF and 8 milliliter of anhydrous toluene, and the resultant solution was cooled at -20°C in a dry ice/methanol bath. To the cooled solution, 5 milliliter of a hexane solution of n-butyllithium (1.6 moles/liter; available from HIROSHIMA WAKO Co., Ltd.) was added, and the resultant solution was stirred at -20°C for 1 hour. Then, 1.4 g of anthraquinone was added, and the resultant solution was stirred at the room temperature for 4 hours and left standing at the room temperature for 12 hours.

The reaction mixture was deactivated with a saturated aqueous solution of ammonium chloride, and the formed solid substance was separated by filtration and washed with methanol.

Then, 2.5 g of 2-bromoterphenyl was dissolved into a mixed solvent of 8 milliliter of anhydrous THF and 8 milliliter of anhydrous toluene, and the resultant solution was cooled at -20°C in a dry ice/methanol bath. To the cooled solution, 5 milliliter of a hexane solution of n-butyllithium (1.6 moles/liter; available from HIROSHIMA WAKO Co., Ltd.) was added, and the resultant solution was stirred at -20°C for 1 hour. Then, the solid substance washed with methanol in the above was added after being dried, and the resultant mixture was stirred at the room temperature for 4 hours and left standing at the room temperature for 12 hours.

The reaction mixture was deactivated with a saturated aqueous solution of ammonium chloride, and a formed solid substance was separated by filtration and washed with methanol. The obtained compound was purified in accordance with the column chromatography, and 1.1 g of a light yellow solid substance was obtained.

Since m/z=598 in FD-MS of the obtained compound, which corresponded to C₄₇H₃₄=598, the compound was identified to be AN5 (the yield: 27%).

### Example 6 (Synthesis of a compound AN7)

Under the atmosphere of argon, a small amount of a solution prepared by dissolving 5 g of 2-bromobiphenyl (available from LANCASTER Company) into 50 milliliter of anhydrous THF was added dropwise to 0.6 g of magnesium. After 0.1 g of iodine was added, the resultant mixture was heated. When the reaction started, the entire amount of the remaining solution was added dropwise at 55 to 60°C, and the mixture was stirred at 50 to 55°C for 2 hours.

Under the atmosphere of argon, 6 g of 2-bromofluorenone was dissolved into 50 milliliter of THF. To the obtained solution, 0.2 g of bis(triphenylphosphine)palladium(II) chloride (available from ALDRICH Company) and 0.6 milliliter of a 1 M toluene solution of diisobutylaluminum hydride (available from ALDRICH Company) were added. After the resultant solution was stirred, the Grignard reagent prepared above was added dropwise over 10 minutes, and the reaction was allowed to proceed at 65°C for one night.

After the reaction was completed, THF was removed by distillation, and the formed crystals were separated by filtration. The crystals were recrystallized from toluene, and 4.9 g of a light yellow powder was obtained.

Under the atmosphere of argon, 4 g of the light yellow powder obtained above was dissolved into a mixed solvent of 8 milliliter of anhydrous THF and 8 milliliter of anhydrous toluene, and the resultant solution was cooled at -20°C in a dry ice/methanol bath. To the cooled solution, 10 milliliter of a hexane solution of n-butyllithium (1.6 moles/liter; available from HIROSHIMA WAKO Co., Ltd.) was added, and the resultant solution was stirred at -20°C for 1 hour. Then, 1.0 g of 9,10-anthraquinone was added, and the resultant solution was stirred at the room temperature for 4 hours and left standing at the room temperature for 12 hours.

The reaction mixture was deactivated with a saturated aqueous solution of ammonium chloride, and the formed solid substance was separated by filtration and washed with methanol.

Then, 2.1 g of 2-bromonaphthalene was dissolved into a mixed solvent of 8 milliliter of anhydrous THF and 8 milliliter of anhydrous toluene, and the resultant solution was cooled at -20°C in a dry ice/methanol bath. To the cooled solution, 10 milliliter of a hexane solution of n-butyllithium (1.6 moles/liter; available from HIROSHIMA WAKO Co., Ltd.) was added, and the resultant solution was stirred at -20°C for 1 hour. Then, the solid substance washed with methanol in the above was added after being dried, and the resultant solution was stirred at the room temperature for 4 hours and left standing at the room temperature for 12 hours.

The reaction mixture was deactivated with a saturated aqueous solution of ammonium chloride, and the formed solid substance was separated by filtration and washed with methanol. The obtained compound was purified in accordance with the column chromatography, and 1.1 g of a light yellow solid substance was obtained.

Since m/z=618 in FD-MS of the obtained compound, which corresponded to C₄₉H₃₀=618, the compound was identified to be AN7 (the yield: 37%).

### Example 7 (Synthesis of a compound AN11)

Under the atmosphere of argon, a small amount of a solution prepared by dissolving 25 g of 2-bromobiphenyl (available from LANCASTER Company) into 50 milliliter of anhydrous THF was added dropwise to 3 g of magnesium. After 0.1 g of iodine was added, the resultant mixture was heated. When the reaction started, the entire amount of the remaining solution was added dropwise at 55 to 60°C, and the mixture was stirred at 50 to 55°C for 2 hours.

Under the atmosphere of argon, 11.5 g of cyclohexanone was dissolved into 50 milliliter of THF. To the obtained solution, 0.2 g of bis(triphenylphosphine)palladium(II) chloride (available from ALDRICH Company) and 3 milliliter of a 1 M toluene solution of diisobutylaluminum hydride (available from ALDRICH Company) were added. After the resultant solution was stirred, the Grignard reagent prepared above was added dropwise over 10 minutes, and the reaction was allowed to proceed at 65°C for one night.

After the reaction was completed, THF was removed by distillation, and the formed crystals were separated by filtration. The crystals were recrystallized from toluene, and 13 g of a white powder was obtained.

The white powder obtained above in an amount of 10 g was suspended into 500 milliliter of purified water, and 0.1 g of ferric chloride monohydrate (available from HIROSHIMA WAKO Co., Ltd.) was added to the resultant suspension. Then, an aqueous solution obtained from 2.5 milliliter of bromine and 200 milliliter of purified water was added dropwise at the room temperature over 1 hour, and the reaction was allowed to proceed at the room temperature for 12 hours.

After the formed crystals were separated by filtration, washed with water and methanol and dissolved into 500 milliliter of chloroform, the resultant solution was washed with an aqueous solution of sodium hydrogen- carbonate and water and dried with anhydrous magnesium sulfate, and the solvent was removed by distillation.

After hexane was added to the resultant mixture, the formed crystals were separated by filtration, and 10 g of a powder was obtained.

Under the atmosphere of argon, 5 g of the powder obtained above was dissolved into a mixed solvent of 8 milliliter of anhydrous THF and 8 milliliter of anhydrous toluene, and the resultant solution was cooled at -20°C in a dry ice/methanol bath. To the cooled solution, 12 milliliter of a hexane solution of n-butyllithium (1.6 moles/liter; available from HIROSHIMA WAKO Co., Ltd.) was added, and the resultant solution was stirred at -20°C for 1 hour. Then, 1.2 g of 9,10-anthraquinone was added, and the resultant solution was stirred at the room temperature for 4 hours and left standing at the room temperature for 12 hours.

The reaction mixture was deactivated with a saturated aqueous solution of ammonium chloride, and the formed solid substance was separated by filtration and washed with methanol. The obtained compound was purified in accordance with the column chromatography, and 2.4 g of a light yellow solid substance was obtained.

Since m/z=642 in FD-MS of the obtained compound, which corresponded to C₅₀H₄₂=642, the compound was identified to be AN11 (the yield: 65%).

### Example 8 (Production of an organic EL device)

A glass substrate (available from GEOMATEC Company) of 25 mm × 75 mm × 1.1 mm thickness having an ITO transparent electrode was cleaned by application of ultrasonic wave in isopropyl alcohol for 5 minutes and then by exposure to ozone generated by ultraviolet light for 30 minutes. The glass substrate having the transparent electrode lines which had been cleaned was attached to a substrate holder of a vacuum vapor deposition apparatus. On the surface of the cleaned substrate at the side having the transparent electrode, a film of N,N'-bis(N,N'-diphenyl-4-aminophenyl)-N,N-diphenyl-4,4'-diamino-1,1'-biphenyl shown below (referred to as a film of TPD232, hereinafter) having a thickness of 60 nm was formed in a manner such that the formed film covered the transparent electrode. The formed film of TPD232 worked as the hole injecting layer. On the formed film of TPD232, a film of N,N,N',N'-tetra (4-biphenyl)diaminobiphenylene shown below (referred to as a film of TBDB, hereinafter) having a thickness of 20 nm was formed. The formed film of TBDB worked as the hole transporting layer. On the formed film of TBDB, a film of AN1 as the light emitting material having a thickness of 40 nm was formed by vapor deposition. At the same time, an amine compound D1 having styryl group which is shown below was vapor deposited as the light emitting material in an amount such that the relative amounts by weight of AN1:D1 was 40:2. The formed film worked as the light emitting layer. On the film formed above, a film of Alq shown below having a thickness of 10 nm was formed. The film of Alq worked as the electron injecting layer. Thereafter, Li (the source of lithium: available from SAES GETTERS Company) as the reducing dopant and Alq were binary vapor deposited, and an Alq:Li film (the thickness: 10 nm) was formed as the electron injecting layer (cathode). On the formed Alq:Li film, metallic aluminum was vapor deposited to form a metal cathode, and an organic EL device was produced.

Using the obtained organic EL device, the efficiency of light emission was measured under application of an electric current with a current density of 10 mA/cm². After the device was stored at 120°C for 500 hours, the condition of the light emitting surface under application of the electric current was observed. The results are shown in Table 1.

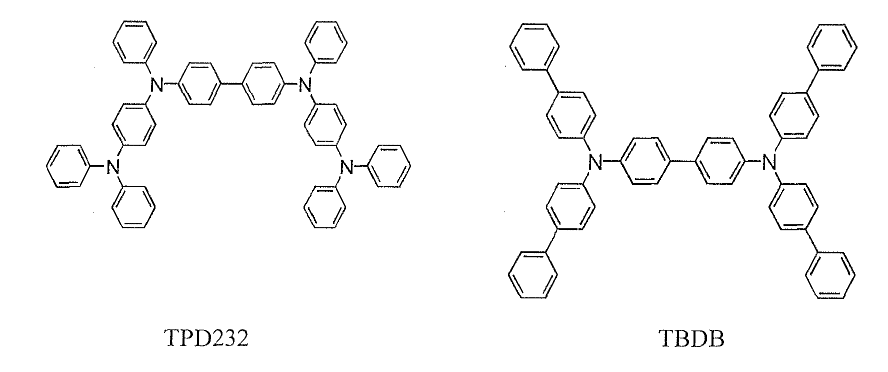

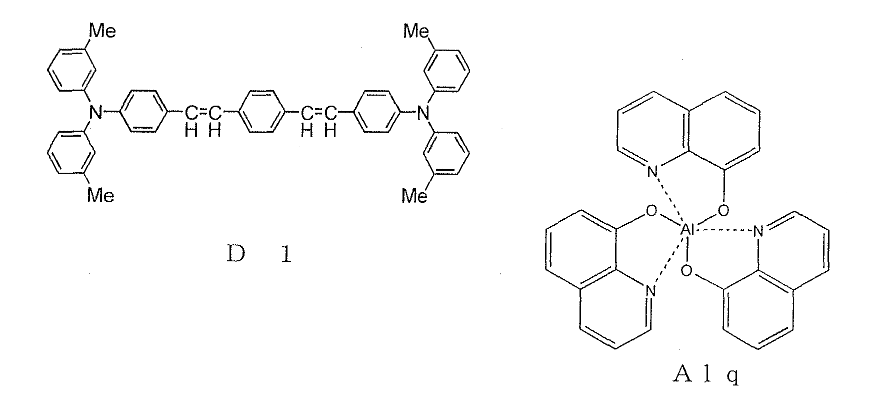

### Example 9 (Production of an organic EL device)

An organic EL device was produced, the efficiency of light emission was measured, and the condition of the light emitting surface was observed in accordance with the same procedures as those conducted in Example 8 except that AN2 was used in place of AN1. The results are shown in Table 1.

### Example 10 (Production of an organic EL device)

An organic EL device was produced, the efficiency of light emission was measured, and the condition of the light emitting surface was observed in accordance with the same procedures as those conducted in Example 8 except that AN3 was used in place of AN1. The results are shown in Table 1.

### Example 11 (Production of an organic EL device)

An organic EL device was produced, the efficiency of light emission was measured, and the condition of the light emitting surface was observed in accordance with the same procedures as those conducted in Example 8 except that AN4 was used in place of AN1. The results are shown in Table 1.

### Example 12 (Production of an organic EL device)

An organic EL device was produced, the efficiency of light emission was measured, and the condition of the light emitting surface was observed in accordance with the same procedures as those conducted in Example 8 except that AN5 was used in place of AN1. The results are shown in Table 1.

### Example 13 (Production of an organic EL device)

An organic EL device was produced, the efficiency of light emission was measured, and the condition of the light emitting surface was observed in accordance with the same procedures as those conducted in Example 8 except that AN7 was used in place of AN1. The results are shown in Table 1.

### Example 14 (Production of an organic EL device)

An organic EL device was produced, the efficiency of light emission was measured, and the condition of the light emitting surface was observed in accordance with the same procedures as those conducted in Example 8 except that AN11 was used in place of AN1. The results are shown in Table 1.

### Comparative Example 1 (Production of an organic EL device)

An organic EL device was produced, the efficiency of light emission was measured, and the condition of the light emitting surface was observed in accordance with the same procedures as those conducted in Example 8 except that an1 shown below was used in place of AN1. The results are shown in Table 1.

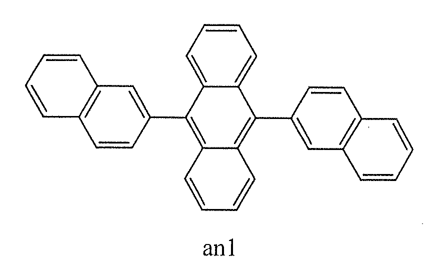

### Comparative Example 2 (Production of an organic EL device)

An organic EL device was produced, the efficiency of light emission was measured, and the condition of the light emitting surface was observed in accordance with the same procedures as those conducted in Example 8 except that an2 expressed by the formula shown below was used in place of AN1. The results are shown in Table 1.

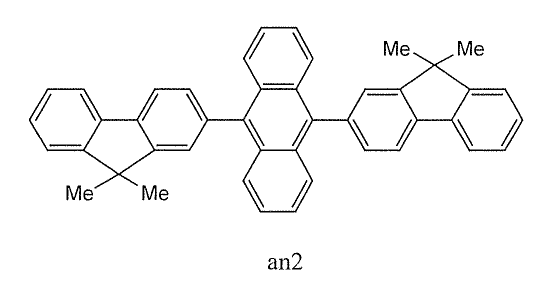

### Comparative Example 3 (Production of an organic EL device)

An organic EL device was produced, the efficiency of light emission was measured, and the condition of the light emitting surface was observed in accordance with the same procedures as those conducted in Example 8 except that an3 shown below was used in place of AN1. The results are shown in Table 1.

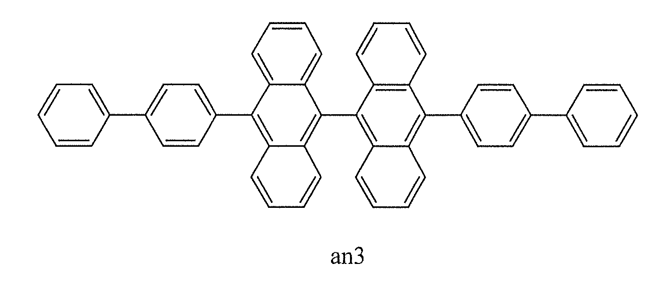

### Comparative Example 4 (Production of an organic EL device)

An organic EL device was produced, the efficiency of light emission was measured, and the condition of the light emitting surface was observed in accordance with the same procedures as those conducted in Example 8 except that an4 shown below was used in place of AN1. The results are shown in Table 1.

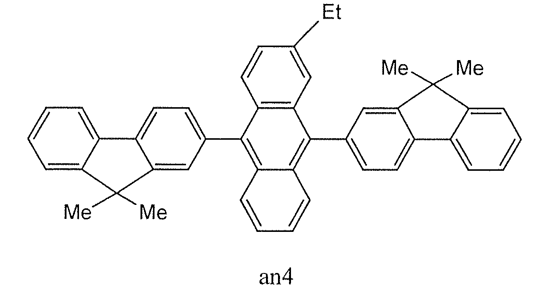

### Comparative Example 5 (Production of an organic EL device)

An organic EL device was produced, the efficiency of light emission was measured, and the condition of the light emitting surface was observed in accordance with the same procedures as those conducted in Example 8 except that an5 shown below was used in place of AN1. The results are shown in Table 1.

During the vapor deposition of an5, the temperature of the vapor deposition boat was elevated to a temperature of 400°C or higher, and the vapor deposition of an5 proceeded while thermal decomposition took place. This phenomenon was estimated based on the peaks of low molecular weight substances observed in accordance with the mass analysis using an apparatus disposed at the inside of the vacuum chamber.

The obtained organic EL device did not emit blue light but whitish blue light due to the effect of the contamination with impurities formed by the decomposition.

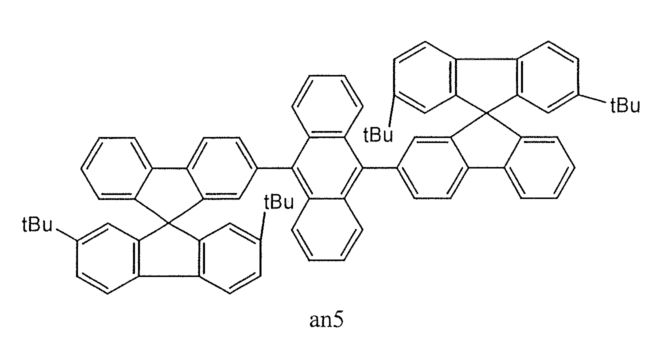

**Table 1**

| | Compounds of light emitting layer | Efficiency of light emission (cd/A) | Situation of light emitting surface after storage at 120°C for 500 hours |
|---|---|---|---|
| Example 8 | AN1/D1 | 9.7 | blue uniform light emission |
| Example 9 | AN2/D1 | 11.0 | blue uniform light emission |
| Example 10 | AN3/D1 | 10.1 | blue uniform light emission |
| Example 11 | AN4/D1 | 10.5 | blue uniform light emission |
| Example 12 | AN5/D1 | 9.6 | blue uniform light emission |
| Example 13 | AN7/D1 | 10.2 | blue uniform light emission |
| Example 14 | AN11/D1 | 10.7 | blue uniform light emission |
| Comparative Example 1 | an1/D1 | 9.0 | appearance of bright spots due to crystal defects |
| Comparative Example 2 | an2/D1 | 8.8 | appearance of bright spots due to crystal defects |
| Comparative Example 3 | an3/D1 | 9.8 | appearance of bright spots due to crystal defects |
| Comparative Example 4 | an4/D1 | 9.0 | appearance of bright spots due to crystal defects |
| Comparative Example 5 | an5/D1 | 8.2 | whitish blue light emission |

As shown in Table 1, the organic EL devices of Examples 8 to 14 exhibited more excellent efficiencies of light emission than those of the devices of Comparative Examples 1 to 5, and uniform blue light could emit even when the devices had been driven for a long time at high temperatures.

In contrast, the crystallization took place in an1 to an3 used in Comparative Examples 1 to 3 since these compounds were highly symmetric.

Although a substituent was introduced into the anthracene ring of an4 used in Comparative Example 4 and the symmetry was relatively low, the decrease in the symmetry was insufficient, and the crystallization took place. It was made clear that a substituent having at least 4 carbon atoms was necessary for preventing the crystallization.

Since an5 used in Comparative Example 5 had spirofluorene group which was a bulky substituent, the crystallization could be prevented although the compound was highly symmetric. However, the temperature of vapor deposition of this anthracene derivative having the bulky substituent at two positions was elevated, and thermal decomposition took place. Therefore, the anthracene derivative having spirofluorenyl group at two positions was not suitable at least for an organic EL device produced in accordance with the vapor deposition process.

Although AN7 used in Example 13 had spirofluorenyl group having a great molecular weight, the spirofluorenyl group was introduced into a single position alone. No thermal decomposition took place unlike the case of Comparative Example 5, and the vapor deposition could be conducted at a temperature of 400°C or lower. Therefore, the uniform emission of blue light was achieved, and the efficiency of light emission was greater than that of Comparative Example 5.

Although AN11 used in Example 14 had the spiro skeleton structure, the structure had a molecular weight smaller than that of spirofluorenyl group. The thermal decomposition did not take place unlike the case of Comparative Example 5 even though the compound had the substituent having the spiro skeleton structure at two positions, and the vapor deposition could be conducted at a temperature of 400°C or lower. Therefore, the uniform emission of blue light was achieved, and the efficiency of light emission was greater than that of Comparative Example 5.

### Example 15

An organic EL device was produced, the efficiency of light emission was measured, and the condition of the light emitting surface was observed in accordance with the same procedures as those conducted in Example 8 except that D2 shown below was used as the light emitting material in place of D1.

Although the efficiency of light emission was 5.0 cd/A, pure blue light was emitted. Uniform light emission was kept at the light emitting surface after storage at 120°C for 500 hours.

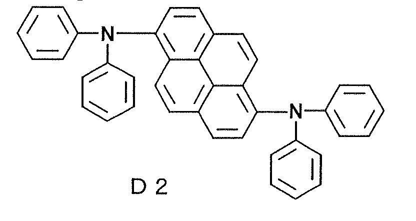

### INDUSTRIAL APPLICABILITY

As described above in detail, the organic EL device utilizing the anthracene derivative of the present invention exhibits a great efficiency of light emission, enables uniform light emission even after the device is driven at a high temperature for a long time and is advantageously used as the device which can be used at high temperatures.

## Claims

1. An anthracene derivative represented by following general formula (1): 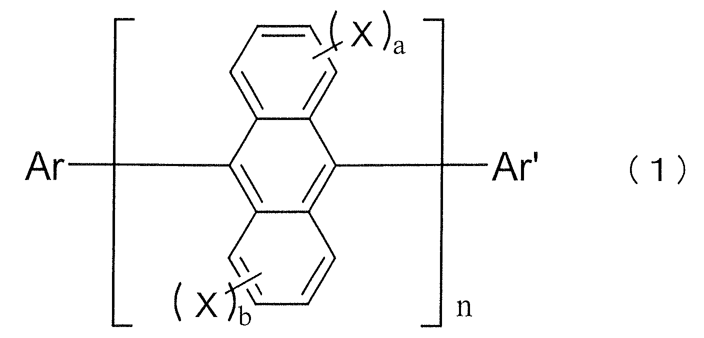 wherein
Ar represents a substituted or unsubstituted group represented by following general formula (2): 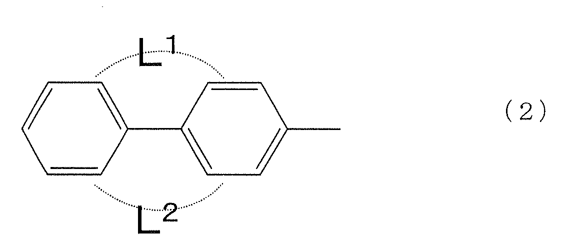 in general formula (2), L¹ and L² each representing a substituted or unsubstituted linking group which forms a cyclic structure, and at least one of the groups represented by L¹ and L² being present,
Ar' represents a substituted or unsubstituted aryl group having 6 to 50 nuclear carbon atoms,
X represents a substituted or unsubstituted alkyl group having 1 to 50 carbon atoms, a substituted or unsubstituted alkoxyl group having 1 to 50 carbon atoms, a substituted or unsubstituted cycloalkyl group having 5 to 50 carbon atoms, a substituted or unsubstituted aralkyl group having 6 to 60 carbon atoms, a substituted or unsubstituted aryl group having 6 to 50 nuclear carbon atoms, a substituted or unsubstituted aromatic heterocyclic group having 5 to 50 nuclear atoms, a substituted or unsubstituted aryloxyl group having 5 to 50 nuclear atoms or a substituted or unsubstituted arylthio group having 5 to 50 nuclear atoms,
a and b each represent an integer of 0 to 4 and when a plurality of groups represented by X are present, they may be the same with or different from each other, and
n represents an integer of 1 to 3 and, when n represents 2 or 3, a plurality of groups represented by: 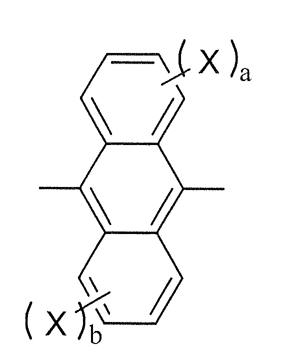
may be a same with or different from each other;
with proviso that
when Ar represents a group represented by a following general formula (3): 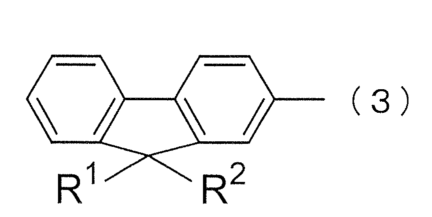 wherein R¹ and R² each represent hydrogen atom, a substituted or unsubstituted alkyl group having 1 to 6 carbon atoms, a substituted or unsubstituted alkoxyl group having 1 to 6 carbon atoms or a substituted or unsubstituted phenyl group,
(i) Ar' represents an aryl group represented by following general formula (4): 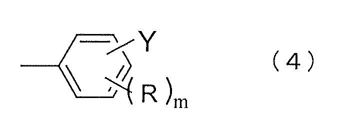 wherein Y represents a substituted or unsubstituted aromatic condensed cyclic residue group having 10 or more nuclear atoms or a substituted or unsubstituted aromatic non-condensed cyclic residue group having 12 or more nuclear atoms, R represents a substituted or unsubstituted alkyl group having 1 to 50 carbon atoms, a substituted or unsubstituted alkoxyl group having 1 to 50 carbon atoms, a substituted or unsubstituted aryl group having 6 to 50 nuclear carbon atoms, a substituted or unsubstituted aromatic heterocyclic group having 5 to 50 nuclear atoms, a substituted or unsubstituted aryloxyl group having 5 to 50 nuclear atoms or a substituted or unsubstituted arylthio group having 5 to 50 nuclear atoms, and m represents an integer of 0 to 4, or
(ii) at least one of a and b does not represent 0, and X represents a substituted or unsubstituted alkyl group having 4 to 50 carbon atoms, a substituted or unsubstituted alkoxyl group having 4 to 50 carbon atoms, a substituted or unsubstituted cycloalkyl group having 5 to 50 carbon atoms, a substituted or unsubstituted aralkyl group having 6 to 60 carbon atoms, a substituted or unsubstituted aryl group having 10 to 50 nuclear carbon atoms, a substituted or unsubstituted aromatic heterocyclic group having 10 to 50 nuclear atoms, a substituted or unsubstituted aryloxyl group having 5 to 50 nuclear atoms or a substituted or unsubstituted arylthio group having 5 to 50 nuclear atoms, and
when Ar represents a group represented by a following general formula (3'): 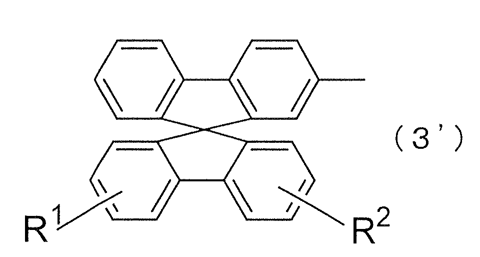
wherein R¹ and R² are as defined above, Ar' represents an aryl group represented by the foregoing general formula (4).

2. An anthracene derivative according to Claim 1, which is a light emitting material for organic electroluminescence devices.

3. An anthracene derivative according to Claim 1, which is a hole transporting material for organic electroluminescence devices.

4. An organic electroluminescence device which comprises a cathode, an anode and one or more organic thin film layer comprising at least one layer including a light emitting layer and sandwiched between the cathode and the anode, wherein at least one layer in the organic thin film layers comprises an anthracene derivative represented by general formula (1) described in Claim 1 singly or as a component of a mixture.

5. An organic electroluminescence device according to Claim 4, wherein said light emitting layer comprises the anthracene derivative represented by general formula (1).

6. An organic electroluminescence device according to Claim 4, wherein said light emitting layer comprises the anthracene derivative represented by general formula (1) as a main component.

7. An organic electroluminescence device according to Claim 4, wherein said light emitting layer further comprises an arylamine compound.

8. An organic electroluminescence device according to Claim 4, wherein said light emitting layer further comprises a styrylamine compound.

9. An organic electroluminescence device according to Claim 4, wherein said organic thin film layers comprise a hole transporting layer, and the hole transporting layer comprises the anthracene derivative represented by general formula (1) described in Claim 1 singly or as a component of a mixture.

10. An organic electroluminescence device according to Claim 9, wherein the hole transporting layer comprises the anthracene derivative represented by general formula (1) as a main component.
